# EUROPEAN PATENT SPECIFICATION

(11) **EP 2 095 760 B1**
(45) Date of publication and mention of the grant of the patent: **25.05.2011**
(21) Application number: 09153927.0
(22) Date of filing: 27.02.2009
(51) Int. Cl.: A61B 3/032, G02B 27/26

(54) **Optotype Presenting Apparatus**
Vorrichtung zur Darstellung von Optotypen
Appareil de présentation d'optotype

(30) Priority: 29.02.2008 JP 2008051330
(43) Date of publication of application: 02.09.2009
(73) Proprietor: Nidek Co., Ltd., Gamagori-shi Aichi 443-0038 (JP)
(72) Inventor: Hirayama, Yukio, Kani-shi Gifu 509-0234 (JP); Oda, Tatefumi, Nukata-gun Aichi 444-0103 (JP); Kanazawa, Yuichiro, Okazaki-shi Aichi 444-3526 (JP)
(74) Representative: Winter, Brandl, Fürniss, Hübner, Röss, Kaiser, Polte - Partnerschaft

(56) References cited:
- DE-A1- 19 947 775
- US-A- 6 011 580
- US-A1- 2006 114 415

## Description

### BACKGROUND OF THE INVENTION

### 1. Field of the Invention

The present invention relates to an optotype presenting apparatus which presents an optotype such as an optotype for a visual acuity test and an optotype for a stereoscopic vision test.

### 2. Description of Related Art

Conventionally, there is known such an optotype presenting apparatus that various optotypes such as optotypes for a visual acuity test are displayed on the screen of a display (see Japanese Patent Application Unexamined Publication No. 2006-42978). In the optotype presenting apparatus comprising the display, when presenting binocular optotypes for a binocular vision test to an examinee, a technique for displaying a three-dimensional image can be used in order to present different optotypes to the right eye and the left eye of the examinee (see Japanese Patent Application Unexamined Publication No. 2002-311385, and Japanese Patent Application Unexamined Publication No. Hei07-322304 corresponding to U.S. Patent No. 5,638,082). More specifically, a sheet-like polarization optical member which comprises two types of linear optical regions oriented in a horizontal direction corresponding to horizontal lines of pixels is arranged in front of the display so that light from the display is converted into light having polarizing axes perpendicular to each other when passing through the polarization optical member. Additionally, polarizing filters which have polarizing axes coinciding with the polarizing axes of the light from the polarization optical member are each placed in front of the right eye and the left eye. Accordingly, different optotypes are presented to the right eye and the left eye, whereby a stereoscopic vision test can be performed.

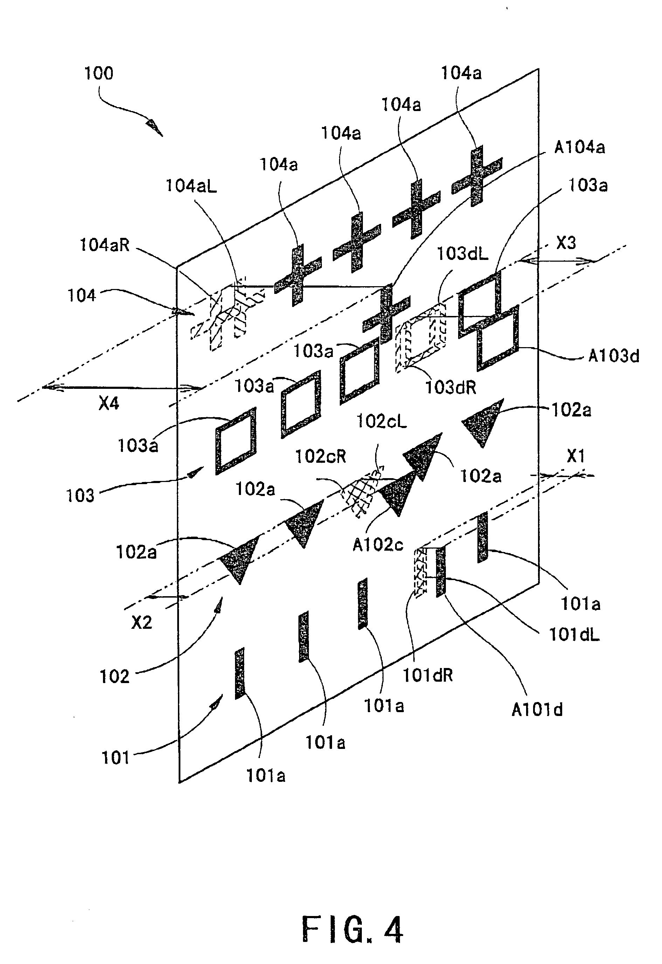

Fig. 4 is a view showing an example of a detailed stereo chart (an optotype for a stereoscopic vision test). A detailed stereo chart 100 shown in Fig. 4 comprises four types of optotypes 101, 102, 103 and 104 having different shapes. In the optotype 101 in the first row from the bottom, vertically long rectangle optotypes 101a in the first, second, third, and fifth columns from the left are reference optotypes to be presented to the right eye and the left eye at the positions on the optotype presenting surface (the display surface of the display) via the polarizing filters. An optotype 101dR is an optotype to be presented only to the right eye via the polarizing filter placed in front of the right eye, while an optotype 101dL is an optotype to be presented only to the left eye via the polarizing filter placed in front of the left eye. When the examinee sees the two optotypes 101dR and 101dL with the both eyes via the polarizing filters, a fusion optotype A101d appears to float toward the front for a float amount X1 with respect to the other four reference optotypes 101a. The pitch (a deviation amount) in the right/left direction between the optotypes 101dR and 101dL is set corresponding to a test distance L such that the float amount X1 is equivalent to a parallax of 40 seconds for example.

Similarly, in the optotype 102 in the second row from the bottom, an optotype 102cR to be presented only to the right eye and an optotype 102cL to be presented only to the left eye are arranged such that a float amount X2 of a fusion optotype A102c with respect to four reference optotypes 102a is equivalent to a parallax of 1 minute for example. In the optotype 103 in the third row from the bottom, an optotype 103cR to be presented only to the right eye and an optotype 103cL to be presented only to the left eye are arranged such that a float amount X3 of a fusion optotype A103c with respect to four reference optotypes 103a is equivalent to a parallax of 2 minutes for example. In the optotype 104 in the top row, an optotype 104aR to be presented only to the right eye and an optotype 104aL to be presented only to the left eye are arranged such that a float amount X4 of a fusion optotype A104a with respect to four reference optotypes 104a is equivalent to a parallax of 4 minutes for example.

If a binocular optotype such as an optotype for a stereoscopic vision test is displayed on a display using a technique of arranging the optical regions of the polarization optical member in the form of a lateral line with respect to the display (see Japanese Patent Application Unexamined Publication No. 2002-311385, and Japanese Patent Application Unexamined Publication No. Hei07-322304), leakage light occurs from the optical regions arranged alternately due to the difference in height of eyes. Accordingly, the examinee cannot see the optotypes separately by the right eye and the left eye, and thus the binocular vision test cannot be performed with accuracy. The occurrence of the above problem can be minimized if the optical regions of the polarization optical member are arranged to have a longitudinal line shape, because eye positions in the lateral direction among different examinees do not significantly differ.

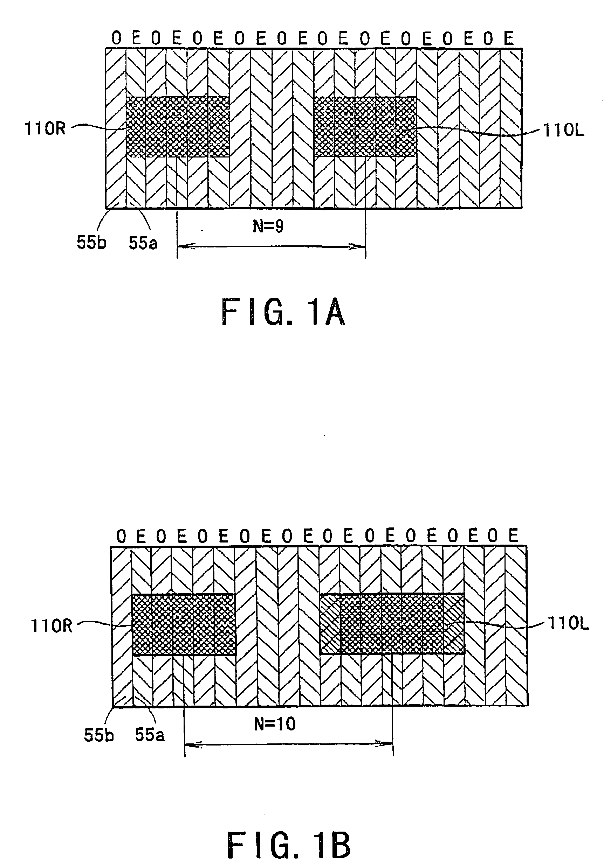

However, it is newly found that the following problem occurs when the optical regions of the polarization optical member are in the form of a longitudinal line. Fig. 1 illustrates how to display an optotype for a stereoscopic vision test for right eye and an optotype for a stereoscopic vision test for left eye to form a fusion optotype A (e.g. the fusion optotype A101d shown in Fig. 4) which has a sink amount or float amount equivalent to an intended parallax with respect to the display surface of the display at a predetermined test distance when the optical regions of the polarization optical member are arranged in the form of a longitudinal line. In Fig. 1, light from odd-numbered columns O is blocked by the polarization optical member and the polarizing filter for left eye of the polarization spectacles and is recognized by the left eye in black, and only light from even-numbered columns E reaches the left eye. The light from the even-numbered columns E is blocked by the polarization optical member and the polarizing filter for right eye of the polarization spectacles and is recognized by the right eye in black, and only the light from the odd-numbered columns O reaches the right eye. An optotype for right eye 110R and an optotype for left eye 110L need to be displayed in the same shape, size, and color so that they appear to fuse into one optotype when seen by the both eyes.

In Fig. 1, the right eye optotype 110R and the left eye optotype 110L are displayed in black using five columns of the optical regions in the form of a longitudinal line of the polarization optical member. Changing the pitch (the deviation amount) between the centers of the right eye optotype 110R and the left eye optotype 110L changes the parallax and thus the float amount of the fusion optotype A. If the pitch N defining the deviation amount between the right eye optotype 110R and the left eye optotype 110L is set to be an odd number of nine columns (nine dots of pixels) in order that the fusion optotype A has a certain parallax, the right eye optotype 110R and the left eye optotype 110L are displayed in five columns (five dots of pixels) and has the same shape.

Fig. 1B shows an example where the pitch N between the right eye optotype 110R and the left optotype 110L is set to be an even number of ten columns (ten dots of pixels) in order that the fusion optotype A has a certain parallax. Here, even if the left eye optotype 110L is formed in five columns, odd-numbered columns O of the optical regions of the polarization optical member, which are not recognized by the left eye, are present on the both sides, and thus the left eye optotype 110L is recognized by the left eye as an optotype in seven columns. Accordingly the right and left eye optotypes 110R and 110L cannot be presented to the both eyes in the same size, failing to appear to fuse into one image when seen by the both eyes. If the pitch N is set to be nine or eleven columns in order to present the right and left eye optotypes 110R and 110L to the both eyes in the same size, the difference between the intended parallax and the actual parallax becomes large, preventing the stereoscopic vision test from being performed with accuracy.

Document US-A-2006/0114415 relates to an optotype presenting apparatus comprising a display, display control means to control the display to display a stereoscopic vision test, a polarization optical member having first and second optical regions in a form of a longitudinal line which are alternately arranged in a lateral direction, the polarization optical member being arranged to convert light from the display into light having polarizing axes perpendicular to each other, the display control means being arranged to control the display to display a first optotype for the right eye and a first optotype for the left eye and a second optotype for the right eye and a second optotype for the left eye.

### SUMMARY OF THE INVENTION

An object of the invention is to overcome the problem of the conventional art described above and to provide an optotype presenting apparatus capable of performing a stereoscopic vision test with accuracy.

To achieve the objects and in accordance with the purpose of the present invention, an optotype presenting apparatus comprises a display, display control means arranged to control the display to display various test optotypes including an optotype for a stereoscopic vision test, and a polarization optical member placed in front of the display, which comprises a first optical region and a second optical region which are in a form of a longitudinal line corresponding to a pixel of the display, have a constant width W, are alternately arranged in a lateral direction, and is arranged to convert light from the display into light having polarizing axes perpendicular to each other, characterized in that the display control means is arranged to control the display to display a first optotype for right eye and a first optotype for left eye in a same size with a deviation amount in the lateral direction therebetween being an odd multiple of the width W, so that the first right eye optotype and the first left eye optotype are presented as a reference optotype, and when the first right eye optotype and the first left eye optotype appear to fuse into the reference optotype to an examinee, the reference optotype has one of a predetermined float amount and a predetermined sink amount with respect to a display surface of the display, and that in order to form a fusion optotype having one of a float amount and a sink amount with respect to the reference optotype which is approximate to an intended parallax, a second optotype for right eye and a second optotype for left eye in a same size are displayed in such positions that a deviation amount in the lateral direction therebetween is an odd multiple of the width W and a parallax of the fusion optotype with respect to the reference optotype is approximate to an intended parallax.

Additional objects and advantages of the invention are set forth in the description which follows, are obvious from the description, or may be learned by practicing the invention. The objects and advantages of the invention may be realized and attained by the apparatus in the claims.

### BRIEF DESCRIPTION OF THE DRAWINGS

The accompanying drawings, which are incorporated in and constitute a part of this specification, illustrate embodiments of the present invention and, together with the description, serve to explain the objects, advantages and principles of the invention. In the drawings,
Figs. 1A and 1B are views illustrating a problem in display of an optotype for right eye and an optotype for left eye which form a fusion optotype for a stereoscopic vision test, which occurs when optical regions of a polarization optical member in the form of a longitudinal line are alternately arranged;
Fig. 2 is a schematic external view showing an optotype presenting apparatus according to a preferred embodiment of the present invention;
Fig. 3 is a view showing a schematic configuration of a presenting portion and a schematic block diagram of a control system of the optotype presenting apparatus;
Fig. 4 is a view showing an example of a detailed stereo chart;
Fig. 5 is a view showing an example of a parameter setting screen;
Figs. 6A and 6B are views illustrating cases where a reference optotype is presented as a fusion reference optotype in a stereo chart;
Figs. 7A and 7B are views illustrating how a fusion reference optotype appears to float or sink;
Fig. 8 is a table showing, for each test distance, the respective pitch which makes the parallax of an optotype for a stereoscopic vision test closer to the target parallax;
Figs. 9A and 9B are views showing examples of a stereo chart; and
Figs 10A and 10B are view showing examples of a stereo chart.

### DETAILED DESCRIPTION OF THE PREFERRED EMBODIMENTS

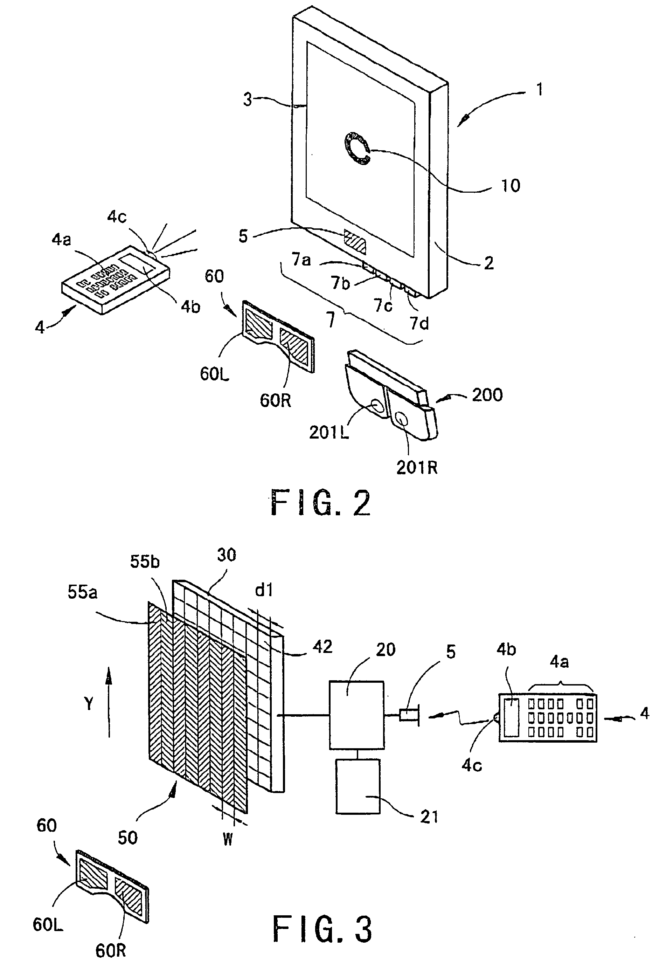

A detailed description of one preferred embodiment of the present invention is provided below with reference to the accompanying drawings. Fig. 2 is a schematic external view showing an optotype presenting apparatus according to a preferred embodiment of the present invention. On the front of a housing 2 of an optotype presenting apparatus 1, an optotype presenting portion 3 is arranged. The presenting portion 3 comprises a screen large enough to display an optotype 10 of a given size such as an optotype for a visual acuity test and an optotype for a binocular vision test even when the presenting portion 3 is placed at a far test distance, e.g., a distance of 5 m from an examinee. In the presenting portion 3, a liquid crystal color display 30 is arranged. As the display 30, a 19- or 17-inch display having a resolution of SXGA is preferably used. In addition, the housing 2 is preferably shaped thinly so as to be hanged on a wall.

At a lower portion on the front surface of the housing 2, a receiving portion 5 is provided which receives a communication signal as infrared light from a remote control 4. In addition, function switches 7 comprising four switches 7a to 7d are arranged on a lower portion of the housing 2 of the optotype presenting apparatus 1. The function switches 7 are used as an operating portion when displaying a condition setting screen on the presenting portion 3 and setting various parameters such as a test distance.

The optotype 10 to be presented on the presenting portion 3 is changed by operating the remote control 4. Polarization spectacles 60 comprising polarizing filters 60L and 60R which have polarizing axes perpendicular to each other are placed in front of the right and left eyes of the examinee at the time of a stereoscopic vision test. The polarizing filter 60L for left eye has the polarizing axis oriented in the direction of 45 degrees, and the polarizing filter 60R for right eye has the polarizing axis oriented in the direction of 135 degrees. If a subjective eye refractive power measurement apparatus (a phoropter) 200 in which corrective lenses such as spherical lenses are switched to be placed in right and left test windows is used for a refractive power test, polarizing filters having polarizing axes perpendicular to each other are switched and placed in the right and left test windows 201R and 201L at the time of a stereoscopic vision test as in the case of the polarization spectacles 60.

Fig. 3 is a view showing a schematic configuration of the presenting portion 3 and a schematic block diagram of a control system of the optotype presenting apparatus 1. The presenting portion 3 comprises the color liquid crystal display 30 and a sheet-like polarization optical member 50 placed in front of the display 30. The polarization optical member 50 is large enough to cover at least the optotype presenting surface on the display 30. The display 30 and the receivingportion 5 are connected to a control unit 20. The control unit 20 comprises a memory 21 which stores various optotype patterns, a decoder circuit which decodes a command signal from the remote control 4, and other constituent elements. When a command signal for switching optotypes or other signals from the remote control 4 is inputted to the control unit 20, the control unit 20 performs display control of pixels 42 of the display 30.

The remote control 4 comprises a plurality of switches 4a for selecting an optotype to be displayed on the display 30, a liquid crystal display portion 4b which displays the selected optotype and the condition of operation by the switches 4a, and a transmitting portion 4c which transmits a signal for switching as infrared light.

Next, the configuration of the polarization optical member 50 is described below. Fig. 3 shows that linearly polarized light having a polarizing axis (a polarizing plane) in a predetermined direction (the vertical direction, the horizontal direction, or an oblique direction of 45 degrees) is emitted from the display 30. In the preferred embodiment of the present invention, light having a polarizing axis in the vertical direction is emitted. The polarization optical member 50 comprises two types of first optical region 55a and second optical region 55b in the form of a line extending in the longitudinal direction (the up/down direction: the Y direction in Fig. 3) corresponding to the size of the pixels 42 of the display 30 which are alternately arranged in a lateral direction. The optical regions 55a and 55b have functions of converting light from the display 30 into linearly polarized light having polarizing axes perpendicular to each other when transmitting the light. In the preferred embodiment of the present invention, a member having a function equivalent to that of a half wavelength plate which makes a phase difference is used as the polarization optical member 50. As is well known, the half wavelength plate is arranged to rotate a vibration direction of incident light by 2 × θ degrees when the incident light enters the half wavelength plate with a polarization plane at θ degree (s) with respect to a fast axis (or a slow axis) of the half wavelength plate. In other words, the half wavelength plate has a function of rotating a direction of the polarizing axis (the vibration direction) of the incident light by inclining a direction of its optically principal axis that is the fast axis (or the slow axis) with respect to the direction of the polarizing axis of the incident light, and has a property of being capable of maintaining light intensity of the incident light.

In Fig. 3, the polarization regions 55a define optical regions for right eye, and the direction of the optically principal axis thereof is arranged so that the incident light is converted into light having a polarizing axis direction which coincides with the polarizing axis direction of 135 degrees of the polarizing filter 60R for right eye of the polarization spectacles 60. The polarization regions 55b define optical regions for left eye, and the direction of the optically principal axis thereof is arranged so that the incident light is converted into light having a polarizing axis direction which coincides with the polarizing axis direction of 45 degrees of the polarizing filter 60L for left eye of the polarization spectacles 60. When looking at the display on the presenting portion 3 through the polarizing filters 60R and 60L placed in front of the right and left eyes, the examinee visually perceives by the left eye only the light from the optical regions 55b which is capable of passing through the polarizing filter 60L, while the light from the optical regions 55a is blocked by the polarizing filter 60L and is not visually perceived by the left eye. In contrast, the examinee visually perceives by the right eye only the light from the optical regions 55a which is capable of passing through the polarizing filter 60R, while the light from the optical regions 55b is blocked by the polarizing filter 60R and is not visually perceived by the right eye. Therefore, different optotypes can be presented to the right eye and the left eye.

If the display 30 is arranged to emit light having a polarizing axis direction of 45 degrees, an optical member which does not have a function of making a phase difference and transmits the light maintaining the polarizing axis direction may be used for the optical regions 55a.

In the present invention, the optical regions 55a and 55b are arranged to cover a region of an integral multiple of the width d1 of the pixel 42.

In addition, in the preferred embodiment of the present invention, a liquid crystal display is used as the display 30. However, a plasma display, an organic EL display, an SED display, and other displays may be used as the display 30. If light exiting from a display other than the liquid crystal display does not have a property of linearly polarized light, a polarizing plate is preferably placed between the display 30 and the polarization optical member 50. In addition, the polarization optical member 50 may be configured such that polarizing plates having a polarizing axis direction of 45 degrees are placed in the optical regions 55a, and polarizing plates having a polarizing axis direction of 135 degrees are placed in the optical regions 55b as described in Japanese Patent Application Unexamined Publication No. Hei07-322304.

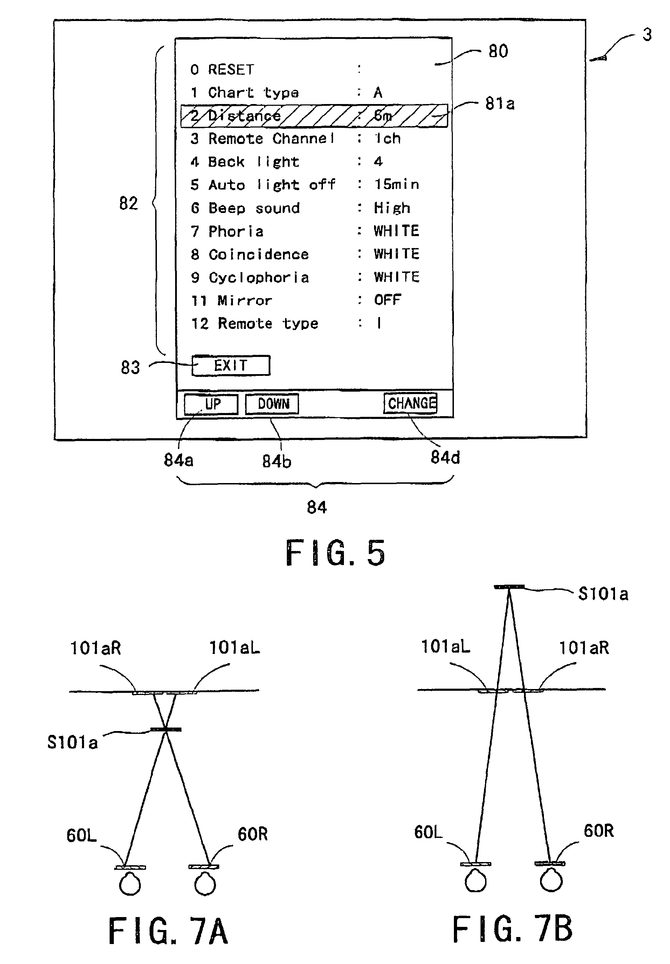

Next, a description of a configuration for changing the setting of a test distance L of the optotype presenting apparatus 1 with respect to the examinee is given. When one of the function switches 7 arranged in the lower portion of the housing 2 of the optotype presenting apparatus 1 is pressed, a parameter setting screen 80 is displayed on the presenting portion 3 as shown in Fig. 5. The parameter setting screen 80 includes items 82 for the setting of various parameters of the optotype presenting apparatus 1. On a lower portion on the parameter setting screen 80, operation conditions 84 are displayed, and an UP key 84a, a DOWN key 84b, and a CHANGE key 84d correspond to the switches 7a, 7b, and 7d of the function switches 7, respectively.

When changing the test distance L, a cursor 81a is moved to "Distance" by operating the UP key 84a and the DOWN key 84b. The "Distance" is a parameter used to set the distance between the examinee's eyes and the presenting portion 3 in accordance with installation conditions of the optotype presenting apparatus 1. Pressing the CHANGE key 84d while the cursor 81a is placed on the "Distance" changes the test distance L in 0.5 m steps from 6 to 3 m (i.e, 6 m, 5.5 m ... 3 m). After the test distance L is set to be a desired value, the cursor 81a is moved to "EXIT" 83 by pressing the DOWN key 84b, and the CHANGE key 84d is pressed to exit the setting screen, thereby completing the setting change of the test distance L. When the setting of the test distance L is changed, the size of the optotype 10 displayed on the display 30 is changed by the control unit 20 corresponding to the test distance L.

A description is given on how to accurately present a stereo chart (an optotype for a stereoscopic vision test) even when the polarization optical member 50 comprising the optical regions 55a and 55b in the form of a longitudinal line which are alternately arranged is used. The stereo chart shown in Fig. 4 is used as an example in the description.

It is assumed that the width W of the optical regions 55a and 55b is equal to the width d1 of the pixel 42 of the display 30 and is 0.294 mm. Here, a test distance L of 3 m means a parallax (a visual angle) of about 20 seconds per dot. As for an optotype 101 shown in Fig. 4, a pitch N between an optotype for right eye 101dR and an optotype for left eye 101dL is determined such that a fusion optotype A101d is formed to have a parallax of 40 seconds with respect to a rectangle reference optotype 101a displayed on the display 30. If the pitch N is set to be three times (an odd multiple) of the width W, it is possible to present the right eye optotype 101dR and the left eye optotype 101dL in the same size, but the parallax is about 60 seconds. If the pitch N is set to be two times (an even multiple) of the width W, the parallax is about 40 seconds and thus the intended parallax is substantially achieved. However, if the pitch N is set to be an even multiple of the width W, it is not possible to present the right eye optotype 101dR and the left eye optotype 101dL in the same size, because of the problem described above.

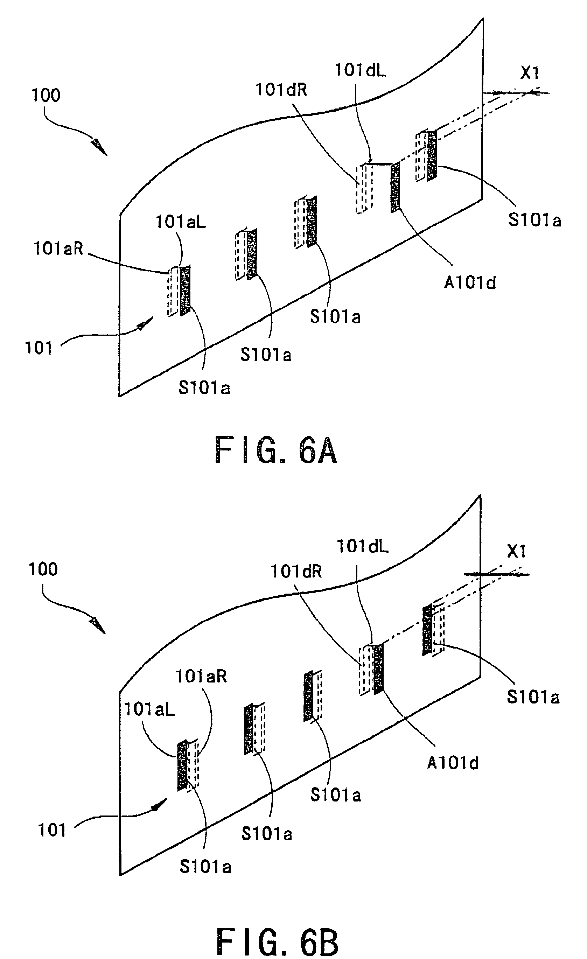

Thus, if the parallax presented when the pitch N is set to be an even multiple of the width W is closer to the intended parallax of 40 seconds compared to the parallax presented when the pitch N is set to be an odd multiple of the width W, optotypes are arranged as follows. An optotype for right eye and an optotype for left eye are displayed on the display 30 such that the reference optotype 101a is presented as a fusion reference optotype S101a which appears to float or sink with respect to the display surface of the display 30, as shown in Figs. 6A and 6B.

In Fig. 6A, in order to form the fusion reference optotype S101a which appears to float with respect to the display surface of the display 30, an optotype 101aR for right eye to be presented only to the right eye via the polarizing filter 60R placed in front of the right eye and an optotype 101aL for left eye to be presented only to the left eye via the polarizing filter 60L placed in front of the left eye are displayed on the display 30. Fig. 7A shows that when the right eye optotype 101aR is placed on the side of the examinee's left eye and the left eye optotype 101aL is placed on the side of the examinee's right eye, the examinee sees the fusion reference optotype S101a with the both eyes and visually perceives it as if it floats with respect to the display surface of the display 30. If a pitch N between the right eye optotype 101aR and the left eye optotype 101aL is set to be N=1 (an odd multiple), the optotypes 101aR and 101aL are presented to the right and left eyes in the same size. When the pitch N is set to be N=1, the parallax is about 20 seconds with respect to the display surface of the display 30. To form a fusion optotype A101d which appears to float with respect to the display surface of the display 30 further than the fusion reference optotype S101a, the pitch N is set to be N=3 (an odd multiple) so that an optotype 101dR for right eye and an optotype 101dL for left eye are presented to the eyes in the same size. Even though the parallax of the fusion optotype A101d is about 60 seconds with respect to the display surface of the display 30, the relative parallax with respect to the fusion reference optotype S101a is 40 seconds (i.e. the difference between the pitches N is an even multiple). Accordingly the parallax can be made closer to the intended parallax of 40 seconds.

Fig. 6B shows a case in which the fusion reference optotype S101a is made to appear to sink with respect to the display surface of the display 30. In order to present the fusion reference optotype S101a such that it appears to sink with respect to the display surface of the display 30, the arrangement of the right eye optotype 101aR and the left eye optotype 101aL is, as shown in Fig. 7B, right-left reversed from the arrangement for floating shown in Fig. 7A. That is, the right eye optotype 101aR is placed on the side of the examinee's right eye and the left eye optotype 101aL is placed on the side of the examinee's left eye. By setting the pitch N between the right eye optotype 101aR and the left eye optotype 101aL to N=1 (an odd multiple) as with the case of floating, they are presented to the right and left eyes in the same size. If the pitch N is N=1, the parallax is about 20 seconds with respect to the display surface of the display 30, and the fusion optotype S101a appears to sink with respect to the display surface. To form the fusion optotype A101d which appears to float with respect to the display surface of the display 30, the pitch N is set to be N=1 (an odd multiple) . Even though the parallax of the fusion optotype A101d with respect to the display surface of the display 30 is about 20 seconds, the relative parallax with respect to the fusion reference optotype S101a is 40 seconds. Accordingly, the intended parallax of 40 seconds can be achieved even when the fusion reference optotype S101a is made to appear to sink.

As for an optotype 102 shown in Fig. 4, the intended parallax of a fusion optotype A102c with respect to a reference optotype 102a is 1 minute (1/60 degrees). If the pitch N is set to be N=3 (an odd multiple), the parallax is about 60 seconds. If the pitch N is set to be N=4 (an even multiple), the parallax is about 1 minute and 20 seconds. In addition, if the pitch N is set to be N=2, the parallax is about 40 seconds. Because the parallax is closest to the intended parallax of 1 minute when the pitch N is set to be N=3 (an odd multiple), the reference optotype 102a remains to be displayed on the display surface of the display 30 as it is, while the fusion optotype A102c is displayed with the pitch N being N=3 (an odd multiple). Accordingly the intended parallax of 1 minute is substantially achieved.

When, for example, the reference optotype 102a is displayed on the display surface of the display 30 such that it is presented to the right eye with a width of 25 columns of optical regions, the reference optotype 102a is presented to the left eye with a width of 27 columns because of the optical regions 55a of the polarization optical member 50 which are present on the both sides of the 25 columns and are not visually perceived by the left eye. However, because the center of the optotype region presented to the right eye coincides with the center of the optotype region presented to the left eye in the display of the reference optotype 102a, no parallax practically occurs when the examinee sees the reference optotype 102a with the both eyes, allowing the examinee to visually perceive the both optotype regions as being substantially identical.

As for an optotype 103 shown in Fig. 4, the intended parallax of a fusion optotype A103d with respect to a reference optotype 103a is 2 minutes. Here, the parallax is closer to the intended parallax when the pitch N is set to be N=6 (an even multiple) comparing to when it is set to be N=5 or N=7 (an odd multiple) . Accordingly, as with the case of the optotype 101, in order to present the reference optotype 103a as a fusion reference optotype S which appears to float (or sink) with respect to the display surface of the display 30, an optotype for right eye and an optotype for left eye which form the fusion reference optotype S are displayed with a pitch N being an odd multiple. If the pitch N of the fusion reference optotype S is set to be N=1 (an odd multiple) and the pitch N of the fusion optotype A103d is set to be N=7 (an odd multiple), the parallax defining the float amount of the fusion optotype A103d with respect to the fusion reference optotype S is 2 minutes, even though the parallax with respect to the display surface of the display 30 is about 2 minutes and 20 seconds. Accordingly, the intended parallax of 2 minutes can be achieved.

As for an optotype 104, the intended parallax of a fusion optotype A104a with respect to a reference optotype 104a is 4 minutes. As with the cases of the optotypes 101 and 103, it is possible to present the fusion optotype A104a with a parallax of about 4 minutes by presenting the reference optotype 104a as a fusion reference optotype S which appears to float (or sink) with respect to the display surface of the display 30.

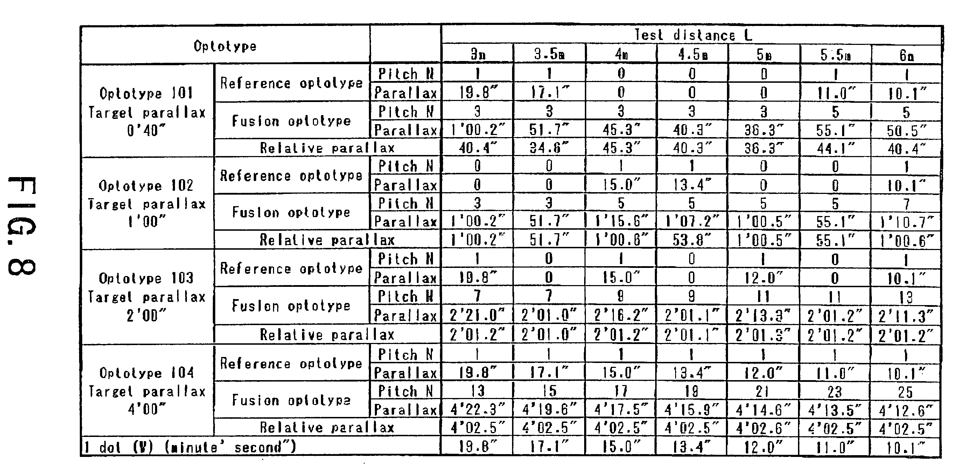

Next, a description of changing the setting of the test distance L is given. With the optotype presenting apparatus 1, the setting of the test distance L can be changed in 0.5 m steps from 3 to 6 m. Fig. 8 is a table showing patterns of pitches N and parallaxes of the optotypes 101, 102, 103 and 104 including the fusion optotypes A each having a float amount equivalent to the target parallax with respect to the respective reference optotypes. Changing the test distance L changes the parallax per dot (the width W of the optical regions 55a and 55b of the polarization optical member 50) . For example, the parallax is about 20 seconds when the test distance L is 3 m, while the parallax is about 17 seconds when the test distance L is 3.5 m. Accordingly, the pitch N of each of the optotypes which makes the parallax closer to the target parallax changes between an even multiple and an odd multiple depending on the test distance L. For example, the pitch N of the optotype 101 which makes the parallax closer to a target parallax of 40 seconds is N=2 (an even multiple) when the test distance L is 3 m or 3.5 m, and is N=3 (an odd multiple) when the test distance L is 4 m. If the test distance L is 4, 4.5 or 5m and accordingly the pitch N is an odd multiple, the target parallax can be substantially achieved by displaying the reference optotype 101a on the display surface of the display 30 as it is and setting the pitch N between the right and left eye optotypes which form the fusion optotype A101d to an odd multiple. If the test distance L is 3, 3.5, 5.5 or 6 m and accordingly the pitch N is an even multiple, the reference optotype is presented as the fusion reference optotype S by setting the pitch N between the right and left eye optotypes to be N=1, and the pitch N between the right and left eye optotypes which form the fusion optotype A101d is accordingly adjusted to an odd multiple so as to make the relative parallax of the fusion optotype A101d which appears to float with respect to the fusion reference optotype S closer to the target parallax.

The above description also applies to the optotypes 102, 103 and 104. As for the optotype 102, the reference optotype 102a is displayed on the display surface of the display 30 as it is if the test distance L is 3, 3.5, 5 or 5.5 m and accordingly the pitch N is an odd multiple. If the test distance L is 4, 4.5 or 6 m and accordingly the pitch N is an even multiple, the reference optotype is presented as the fusion reference optotype S by setting the pitch N between the right and left eye optotypes which form the fusion reference optotype S to be N=1. In order to make the relative parallax of the fusion optotype A102c with respect to the fusion reference optotype S closer to the target parallax, the pitch N between the right and left eye optotypes which form the fusion optotype A102c is adjusted to be an odd multiple. As for the optotypes 103 and 104, if the pitch N for achieving the target parallax is an odd multiple, the pitch N of the reference optotype is set to be N=0 as shown in Fig. 8. If the pitch N is an even multiple, the pitch N of the reference optotype is set to be N=1 in Fig. 8. In any of the optotypes 101 to 104, if the pitch N for achieving the target parallax is an even multiple, the pitch N of the fusion optotype A is adjusted to be the odd multiple indicated in the respective rows of the pitch N of the fusion optotype A in the table shown in Fig. 8.

For each of the optotypes 101, 102, 103 and 104 used to perform a stereoscopic vision test with an intended parallax, the control unit 20 determines whether or not to present the each of reference optotypes (101a, 102a, 103a and 104a) as the fusion reference optotype S for each test distance L as shown in Fig. 8. In addition, the control unit 20 determines the pitch N between the right and left eye optotypes which form the fusion reference optotype S and the pitch N between the right and left eye optotypes which form the fusion optotype A such that they are odd multiples of the width W. In the above determinations, it is preferable if patterns such as those shown in Fig. 8 are prestored in the memory 21. To be more specific, it is preferable if a pattern of displaying the reference optotype on the display surface of the display 30 as it is and a pattern of the pitch N between the right and left eye optotypes which form the fusion reference optotype S which appears to sink or float with respect to the display surface of the display 30 and the pitch N between the right and left eye optotypes which form the fusion optotype A having an intended parallax with respect to the fusion reference optotype S are stored for each of the test distances L in the memory 21. When the setting of the test distance L is changed, the control unit 20 retrieves the patterns of optotype display corresponding to the set test distance L and controls the display of the stereo chart on the display 30 according to the retrieved patterns. Alternatively, instead of prestoring the patterns for each of the test distances L in the memory 21, the control unit 20 may calculate and determine the display pattern of the optotypes which makes the parallax close to the intended parallax and controls the display of the chart on the display 30 based on the calculation result.

Fig. 8 shows a case in which the fusion reference optotype S is presented such that it appears to float when the pitch N is an even multiple; however, it is also preferable to present the fusion reference optotype S such that it appears to sink with respect to the display surface of the display 30 by right-left reversing the arrangement of the right and left eye optotypes. Additionally, the fusion optotype A having an intended parallax with respect to the reference optotype or the fusion reference optotype S may be presented such that it appears to sink.

A description of operations for performing a test using the optotype presenting apparatus 1 having the configuration described above is now given. A test is started after positioning an examinee at a predetermined test distance L. An optotype selecting signal which is transmitted from the remote control 4 by operating the switch 4a is received by the receiving portion 5 of the optotype presenting apparatus 1. The control unit 20 retrieves the corresponding optotype from the memory 21 based on the received signal and controls the display 30 to display the selected optotype. When performing a test using the stereo chart shown in Fig. 4, the polarization spectacles 60 are placed in front of the examinee's eyes before starting the test. Then the control unit 20 controls the display 30 to display the stereo chart 100 according to the patterns corresponding to the set test distance L.

In the test using the stereo chart 100 shown in Fig. 4, the examinee is asked to tell which optotype of the five optotypes in each row appears to float. For example, if the third fusion optotype A102c from the left in the second row from the bottom appears for the examinee to float, the examinee is evaluated to have stereo vision enough to distinguish a parallax of 1 minute. In addition, if the fourth fusion optotype A101d from the left in the first row from the bottom appears for the examinee to float, the examinee is evaluated to have stereo vision enough to distinguish a parallax of 40 seconds. In order to evaluate the degree of examinee's stereo vision in the stereoscopic vision test, it is preferable that the deviation of the parallax of the optotype for a stereoscopic vision test from the target parallax is minimized. The optotype presenting apparatus 1 is capable of presenting an optotype for stereoscopic vision test having a parallax which is made closer to the target parallax (the intended parallax) set for the stereoscopic vision test, and is thus capable of performing an accurate test.

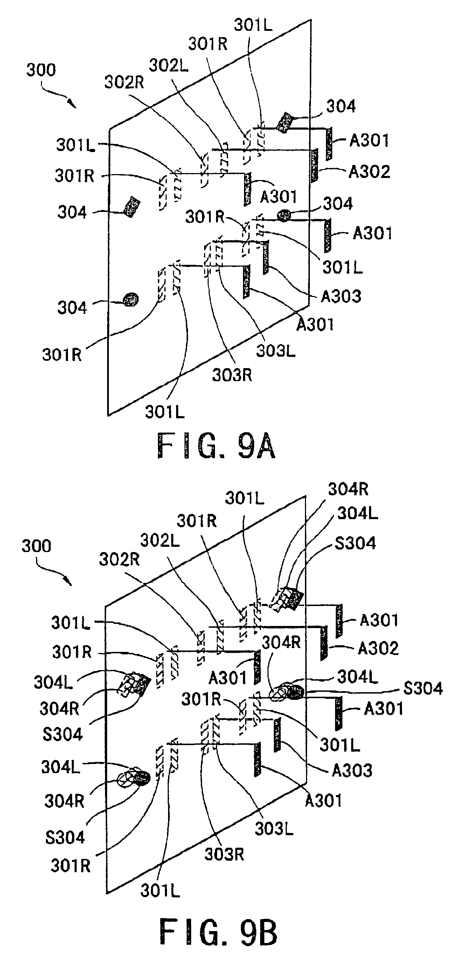

A description of an example of another stereo chart is given. A detailed stereo chart 300 shown in Fig. 9A comprises reference optotypes 304 which are arranged in four positions on the right and left in the upper and bottom rows and are presented in the same size to the both eyes of an examinee, optotypes for right eye 301R and optotypes for left eyes 301L arranged on the right and left in the upper and bottom rows to form four fusion optotypes A301 having a float amount equivalent to a parallax of 10 minutes with respect to the reference optotypes 304, an optotype for right eye 302R and an optotype for left eye 302L arranged in the middle in the upper row to form a fusion optotype A302 having a float amount equivalent to a parallax of 40 seconds with respect to the fusion optotypes A301, and an optotype for right eye 303R and an optotype for left eye 303L arranged in the middle in the bottom row to form a fusion optotype A303 having a sink amount equivalent to a parallax of 40 seconds with respect to the fusion optotypes A301.

In the stereo chart 300, the parallax per dot (the width W) is about 20 seconds when the test distance L is 3 m. When the intended parallax of the fusion optotype A301 is 10 minutes, a pitch N between the right eye optotype 301R and the left eye optotype 301L of N=29 brings a parallax of about 9 minutes and 45 seconds, N=30 brings about 10 minutes and 5 seconds, and N=31 brings about 10 minutes and 25 seconds. Thus, N=30, an even multiple, brings a parallax which is approximate to the intended parallax of 10 minutes.

As for the fusion optotype A302 which appears to float with an intended parallax of 40 seconds with respect to the fusion optotype A301, a pitch N between the right eye optotype 302R and the left eye optotype 302L of N=32 brings a parallax which is approximate to the intended parallax. As for the fusion optotype A303 which appears to sink with an intended parallax of 40 seconds with respect to the fusion optotype A301, a pitch N between the right eye optotype 303R and the left eye optotype 303L of N=28 brings a parallax which is approximate to the intended parallax.

As described above, the pitches N between the right and left eye optotypes which form the fusion optotypes A301 to A303 are even multiples of the width W. In this case, based on the concept described above, the reference optotype 304 is presented as a fusion reference optotype S304 which appears to float or sink with respect to the display surface of the display 30 by, as shown in Fig. 9B, displaying an optotype for right eye 304R and an optotype for left eye 304L which form the fusion reference optotype S304 and setting the pitch N therebetween to N=1, an odd multiple. Accordingly, the pitch N between the right eye optotype 301R and the left eye optotype 301L which form the fusion optotype A301 is set to be N=31, an odd multiple, the pitch N between the right eye optotype 302R and the left eye optotype 302L which form the fusion optotype A302 is set to be N=33, an odd multiple, and the pitch N between the right eye optotype 303R and the left eye optotype 303L which form the fusion optotype A303 is set to be N=29, an odd multiple. Consequently, the parallaxes of the fusion optotypes A301, A302 and A303 are made closer to the respective target parallaxes.

In the example above, the test distance L of the stereo chart shown in Fig. 9 is set to 3 m. When the test distance L is changed, as with the case of the foregoing stereo chart 100, it is determined for each test distance L whether or not to present the reference optotype 304 as the fusion reference optotype S304 which appears to float or sink with respect to the display surface of the display 30. In the case of forming the fusion reference optotype S304, the pitch N between the right and left eye optotypes which form the fusion reference optotype S304 and the pitches N between the right and left eye optotypes which form the fusion optotypes A301, A302 and A303 are set to be odd multiples of the width W.

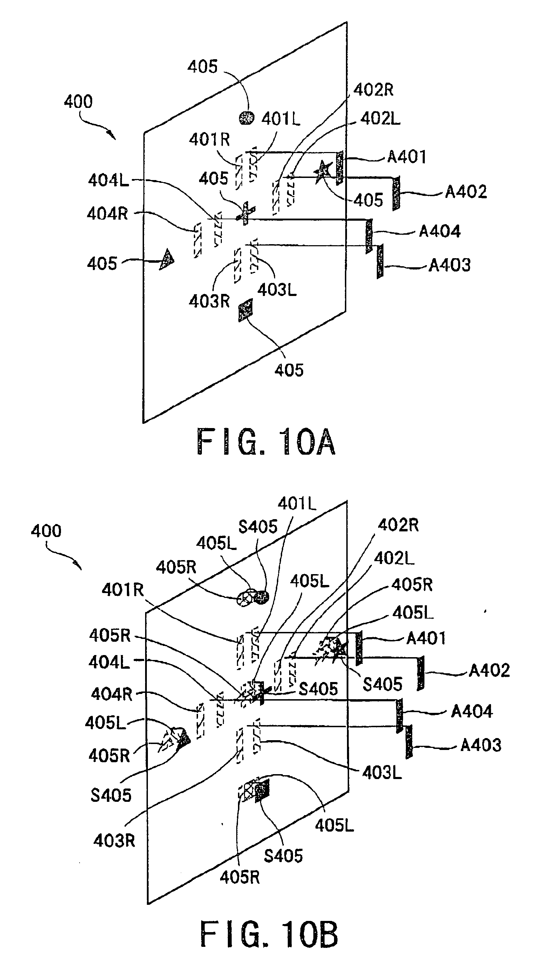

Fig. 10 is a view showing an example of another stereo chart. A stereo chart 400 shown in Fig. 10A comprises reference optotypes 405 which are arranged in five positions in the center, top, bottom, right, and left and are presented to the both eyes of an examinee, an optotype for right eye 401R and an optotype for left eye 401L which form an upper fusion optotype A401 having a float amount equivalent to a parallax of 10 minutes with respect to the reference optotypes 405, an optotype for right eye 402R and an optotype for left eye 402L which form a right fusion optotype A402 having a float amount equivalent to a parallax of 1 minute with respect to the fusion optotype A401 (a parallax of 11 minutes with respect to the reference optotypes 405), an optotype for right eye 403R and an optotype for left eye 403L which form a bottom fusion optotype A403 having a float amount equivalent to a parallax of 2 minutes with respect to the fusion optotype A402 (a parallax of 13 minutes with respect to the reference optotypes 405), and an optotype for right eye 404R and an optotype for left eye 404L which form a left fusion optotype A404 having a float amount equivalent to a parallax of 4 minutes with respect to the fusion optotype A403 (a parallax of 17 minutes with respect to the reference optotype 405).

In the stereo chart 400, the parallax per dot (the width W) is about 20 seconds when the test distance L is 3 m. The pitches N between the right and left eye optotypes which make the parallaxes of the fusion optotypes A401, A402, A403 and A404 with respect to the reference optotype 405 approximate to 10, 11, 13 and 17 minutes are even multiples of N=30, 32, 38 and 50, respectively. Based on the same concept as the foregoing example, the reference optotype 405 is presented as a fusion reference optotype S405 which appears to float or sink with respect to the display surface of the display 30 by, as shown in Fig. 10B, displaying an optotype for right eye 405R and an optotype for left eye 405L which form the fusion reference optotype S405 and setting the pitch N therebetween to be N=1, an odd multiple. Accordingly, the pitch N between the right eye optotype 401R and the left eye optotype 401L which form the fusion optotype A401 is set to be N=31, an odd multiple, the pitch N between the right eye optotype 402R and the left eye optotype 402L which form the fusion optotype A402 is set to be N=33, an odd multiple, the pitch N between the right eye optotype 403R and the left eye optotype 403L which form the fusion optotype A403 is set to be N=39, an odd multiple, and the pitch N between the right eye optotype 404R and the left eye optotype 404L which form the fusion optotype A404 is set to be N=51, an odd multiple. Consequently, the parallaxes of the fusion optotypes A401, A402, A403 and A404 are made closer to the respective target parallaxes. Any change in the test distance L is dealt in the same manner as the stereo chart 300 shown in Fig. 9.

The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

## Claims

1. An optotype presenting apparatus comprising:
a display (30);
display control means (20) arranged to control the display to display various optotypes including an optotype for a stereoscopic vision test (101); and
a polarization optical member (50) placed in front of the display, which comprises a first optical region (55a) and a second optical region (55b) which are in a form of a longitudinal line corresponding to an integral multiple of a width (W) of the pixel of the display, and are alternately arranged in a lateral direction, and the polarization optical member being arranged to convert light from the display into light having polarizing axes perpendicular to each other,
the display control means being arranged to control the display to display a first optotype for right eye (101aR) and a first optotype for left eye (101aL) in a same size with a deviation amount in the lateral direction therebetween being an odd multiple of the width of the pixel (W), and when the first right eye optotype (101aR) and the first left eye optotype (101aL) appear to fuse into a reference optotype (S101a) to eyes of an examinee, the reference optotype (S101a) has one of a predetermined float amount and a predetermined sink amount with respect to a display surface of the display, and
in order to display a fusion optotype (A101 d) having one of a float amount (X1) and a sink amount (X1) with respect to the reference optotype (S101a) which is approximate to an intended parallax, the display control means is arranged to control the display to display a second optotype for right eye (101dR) and a second optotype for left eye (101dL) in a same size so that a deviation amount in the lateral direction therebetween is an odd multiple of the width (W) and a parallax of the fusion optotype (A101d) with respect to the reference optotype (S101a) is approximate to an intended parallax.

2. The optotype presenting apparatus according to claim 1, further comprising distance setting means (7) arranged to input a setting signal of a test distance between the examinee and the display,
wherein, corresponding to a set test distance,
the display control means displays the first right eye optotype (101aR) and the first left eye optotype (101aL) with no deviation amount in the lateral direction therebetween if the parallax of the fusion optotype (A101d) with respect to the display surface is closer to an intended parallax when the deviation amount in the lateral direction between the second right eye optotype (101dR) and the second left eye optotype (101dL) is set to be an odd multiple of the width (W) comparing to when the deviation amount in the lateral direction between the second right eye optotype (101dR) and the second left eye optotype (101dL) is set to be an even multiple of the width (W), and
the display controls means displays the first right eye optotype (101 aR) and the first left eye optotype (101aL) so that the deviation amount in the lateral direction therebetween is an odd multiple of the width (W) and displays the second right eye optotype (101dR) and the second left eye optotype (101dL) so that the deviation amount in the lateral direction therebetween is an odd multiple of the width (W) and the parallax of the fusion optotype (A101d) with respect to the reference optotype (S101a) is approximate to an intended parallax, if the parallax of the fusion optotype (A101d) with respect to the display surface is closer to an intended parallax when the deviation amount in the lateral direction between the second right eye optotype (101dR) and the second left eye optotype (101dL) is set to be an even multiple of the width (W) comparing to when the deviation amount in the lateral direction between the second right eye optotype (101dR) and the second left eye optotype (101dL) is set to be an odd multiple of the width (W).

3. The optotype presenting apparatus according to claim 1, further comprising:
distance setting means (7) arranged to input a setting signal of a test distance between the examinee and the display; and
a memory (21) which stores a pattern of the optotype for the stereoscopic vision test, the memory prestoring, for each of the test distances which can be set by the distance setting means, a first pattern in which the first right eye optotype (101aR) and the first left eye optotype (101aL) are displayed with no deviation amount in the lateral direction therebetween, and the second right eye optotype (101dR) and the second left eye optotype (101dL) are displayed so that the deviation amount in the lateral direction therebetween is an odd multiple of the width (W) and the parallax of the fusion optotype (A101d) with respect to the reference optotype (S101a) is approximate to an intended parallax, and a second pattern in which the first right eye optotype(101aR) and the first left eye optotype (101aL) are displayed so that the deviation amount in the lateral direction therebetween is an odd multiple of the width (W), and the second right eye optotype (101dR) and the second left eye optotype (101dL) are displayed so that the deviation amount in the lateral direction therebetween is an odd multiple of the width (W) and the parallax of the fusion optotype (A101d) with respect to the reference optotype (S101a) is approximate to an intended parallax,
wherein the display control means is arranged to retrieve the pattern stored in the memory based on the test distance set by the distance setting means and to control the display to display the optotype according to the retrieved pattern.

## Patentansprüche

1. Optotypendarstellungsvorrichtung mit:
einer Anzeige (30);
einem Anzeigenansteuerungsmittel (20), das ausgelegt ist, um die Anzeige so anzusteuern, dass sie verschiedene Optotypen einschließlich einer Optotype für einen Stereosehtest (101) anzeigt; und
einem optischen Polarisationselement (50), das vor der Anzeige angeordnet ist und einen ersten optischen Bereich (55a) und einen zweiten optischen Bereich (55b) umfasst, die in Form einer Längslinie ausgebildet sind, die einem ganzzahligen Vielfachen einer Breite (W) des Pixels der Anzeige entspricht, und die in Querrichtung abwechselnd angeordnet sind, wobei das optische Polarisationselement (50) so angeordnet ist, dass es Licht von der Anzeige in Licht umwandelt, dessen Polarisationsachsen senkrecht zueinander sind,
wobei das Anzeigenansteuerungsmittel so angeordnet ist, dass die Anzeige so angesteuert wird, dass sie eine erste Optotype für das rechte Auge (101aR) und eine erste Optotype für das linke Auge (101aL) in gleicher Größe anzeigt, wobei ein Abweichungsbetrag in der Querrichtung dazwischen ein nicht ganzzahliges Vielfaches der Breite (W) des Pixels ist, und wobei, wenn die erste Optotype für das rechte Auge (101aR) und die erste Optotype für das linke Auge (101aL) in den Augen der zu untersuchenden Person in eine Referenzoptotype (S101a) zu verschmelzen scheinen, die Referenzoptotype (S101a) entweder einen vorbestimmten Nach-vorn-Versetzungsbetrag oder einen vorbestimmten Nach-hinten-Versetzungsbetrag bezüglich einer Anzeigeoberfläche der Anzeige hat; und
wobei das Anzeigenansteuerungsmittel, um eine Verschmelzungsoptotype (A101d) anzuzeigen, die bezüglich der Referenzoptotype (S101a) entweder einen Nach-vorn-Versetzungsbetrag (X1) oder einen Nach-hinten-Versetzungsbetrag (X1) hat, der etwa einer Sollparallaxe entspricht, so angeordnet ist, dass die Anzeige so angesteuert wird, dass sie eine zweite Optotype für das rechte Auge (101dR) und eine zweite Optotype für das linke Auge (101dL) in gleicher Größe anzeigt, so dass ein Abweichungsbetrag in der Querrichtung dazwischen ein nicht ganzzahliges Vielfaches der Breite (W) ist und eine Parallaxe der Verschmelzungsoptotype (A101d) bezüglich der Referenzoptotype (S101a) in etwa einer Sollparallaxe entspricht.

2. Optotypendarstellungsvorrichtung nach Anspruch 1, ferner umfassend ein Abstandeinstellungsmittel (7), das ausgelegt ist, um ein Einstellsignal eines Testabstandes zwischen der zu untersuchenden Person und der Anzeige einzugeben,
wobei, entsprechend eines eingestellten Testabstandes
das Anzeigenansteuerungsmittel die erste Optotype für das rechte Auge (101aR) und die erste Optotype für das linke Auge (101aL) ohne Abweichungsbetrag in der Querrichtung dazwischen anzeigt, wenn die Parallaxe der Verschmelzungsoptotype (A101d) bezüglich der Anzeigeoberfläche näher an einer Sollparallaxe ist, wenn der Abweichungsbetrag in der Querrichtung zwischen der zweiten Optotype für das rechte Auge (101dR) und der zweiten Optotype für das linke Auge (101dL) so eingestellt ist, dass er ein nicht ganzzahliges Vielfaches der Breite (W) ist, im Vergleich zu einem Fall, wo der Abweichungsbetrag in der Querrichtung zwischen der zweiten Optotype für das rechte Auge (101dR) und der zweiten Optotype für das linke Auge (101dL) so eingestellt ist, dass er ein ganzzahliges Vielfaches der Breite (W) ist, und
das Anzeigenansteuerungsmittel die erste Optotype für das rechte Auge (101aR) und die erste Optotype für das linke Auge (101aL) so anzeigt, dass der Abweichungsbetrag in der Querrichtung dazwischen ein nicht ganzzahliges Vielfaches der Breite (W) ist, und die zweite Optotype (101dR) für das rechte Auge und die zweite Optotype (101dL) für das linke Auge so anzeigt, dass der Abweichungsbetrag in der Querrichtung dazwischen ein nicht ganzzahliges Vielfaches der Breite (W) ist und die Parallaxe der Verschmelzungsoptotype (A101d) bezüglich der Referenzoptotype (S101a) ist etwa einer Sollparallaxe entspricht, falls die Parallaxe der Verschmelzungsoptotype (A101 d) bezüglich der Anzeigeoberfläche näher bei einer Sollparallaxe ist, wenn der Abweichungsbetrag in der Querrichtung zwischen der zweiten Optotype des rechten Auges (101dR) und der zweiten Optotype für das linke Auge (101dL) so eingestellt ist, dass er ein ganzzahliges Vielfaches der Breite (W) ist, im Vergleich zu einem Fall, wo der Abweichungsbetrag in der Querrichtung zwischen der zweiten Optotype für das rechte Auge (101dR) und der zweiten Optotype für das linke Auge (101dL) so eingestellt ist, dass er ein nicht ganzzahliges Vielfaches der Breite (W) ist.

3. Optotypendarstellungsvorrichtung nach Anspruch 1, ferner umfassend:
ein Abstandeinstellungsmittel (7), das dazu ausgelegt ist, ein Einstellsignal eines Testabstandes zwischen der zu untersuchenden Person und der Anzeige einzugeben; und
einen Speicher (21), der ein Muster der Optotype für den Stereosehtest speichert, wobei der Speicher für jeden der Testabstände, der durch das Abstandeinstellungsmittel eingestellt werden kann, ein erstes Muster vorspeichert, in dem die erste Optotype für das rechte Auge (101aR) und die erste Optotype für das linke Auge (101aL) ohne Abweichungsbetrag dazwischen angezeigt werden und die zweite Optotype für das rechte Auge (101dR) und die zweite Optotype für das linke Auge (101dL) so angezeigt werden, dass der Abweichungsbetrag in der Querrichtung dazwischen ein nicht ganzzahliges Vielfaches der Breite (W) ist und die Parallaxe der Verschmelzungsoptotype (A101d) bezüglich der Referenzoptotype (S101a) in etwa eine Sollparallaxe ist, und ein zweites Muster vorspeichert, in dem die erste Optotype für das rechte Auge (101 aR) und die erste Optotype für das linke Auge (101 aL) so angezeigt werden, dass der Abweichungsbetrag in der Querrichtung dazwischen ein nicht ganzzahliges Vielfaches der Breite (W) ist, und die zweite Optotype für das rechte Auge (101 dR) und die zweite Optotype für das linke Auge (101 dL) so angezeigt werden, dass der Abweichungsbetrag in der Querrichtung dazwischen ein nicht ganzzahliges Vielfaches der Breite (W) ist und die Parallaxe der Verschmelzungsoptotype (A101d) bezüglich der Referenzoptotype (S101a) in etwa eine Sollparallaxe ist,
wobei das Anzeigensteuerungsmittel ausgelegt ist, um das in dem Speicher gespeicherte Muster auf der Grundlage des durch das Abstandeinstellungsmittel eingestellten Testabstandes abzurufen und die Anzeige so zu steuern, dass sie die Optotype entsprechend dem abgerufenen Muster anzeigt.

## Revendications

1. Appareil de présentation d'optotype comprenant :
un écran d'affichage (30) ;
un moyen de commande d'affichage (20) agencé pour commander l'affichage afin d'afficher différents optotypes comprenant un optotype pour un test de vision stéréoscopique (101) ; et
un élément optique de polarisation (50) placé devant l'écran d'affichage, qui comprend une première région optique (55a) et une seconde région optique (55b) qui se présentent sous la forme d'une ligne longitudinale correspondant à un entier multiple d'une largeur (W) du pixel de l'affichage, et qui sont agencées en alternance dans une direction latérale, et l'élément optique de polarisation étant agencé pour convertir la lumière provenant de l'écran d'affichage en lumière ayant des axes de polarisation perpendiculaires les uns aux autres,
le moyen de commande d'affichage étant agencé pour commander l'affichage afin d'afficher un premier optotype pour l'oeil droit (101aR) et un premier optotype pour l'oeil gauche (101aL) à une même taille, un degré d'écart dans la direction latérale entre les deux étant un multiple impair de la largeur (W) du pixel, et lorsque le premier optotype d'oeil droit (101aR) et le premier optotype d'oeil gauche (101aL) semblent fusionner en un optotype de référence (S101a) vers les yeux d'une personne examinée, l'optotype de référence (S101a) présente un degré de flottement prédéfini ou un degré d'enfoncement prédéfini par rapport à une surface d'affichage de l'écran d'affichage, et
afin d'afficher un optotype de fusion (A101d) ayant un degré de flottement (X1) ou un degré d'enfoncement (X1) par rapport à l'optotype de référence (S101a) qui est proche d'un parallaxe prévu, le moyen de commande d'affichage est agencé pour commander l'affichage afin d'afficher un second optotype pour l'oeil droit (101dR) et un second optotype pour l'oeil gauche (101dL) à une même taille de sorte qu'un degré d'écart dans la direction latérale entre les deux soit un multiple impair de la largeur (W) et qu'un parallaxe de l'optotype de fusion (A101d) par rapport à l'optotype de référence (s101a) soit proche d'un parallaxe prévu.

2. Appareil de présentation d'optotype selon la revendication 1, comprenant en outre un moyen de réglage de distance (7) agencé pour entrer un signal de réglage d'une distance test entre la personne examinée et l'écran d'affichage,
dans lequel, en correspondance avec une distance test réglée,
le moyen de commande d'affichage affiche le premier optotype d'oeil droit (101aR) et le premier optotype d'oeil gauche (101aL) sans aucun degré d'écart dans la direction latérale entre les deux si le parallaxe de l'optotype de fusion (A101d) par rapport à la surface d'affichage est plus proche d'un parallaxe prévu lorsque le degré d'écart dans la direction latérale entre le second optotype d'oeil droit (101dR) et le second optotype d'oeil gauche (101dL) est réglé pour être un multiple impair de la largeur (W) que lorsque le degré d'écart dans la direction latérale entre le second optotype d'oeil droit (101dR) et le second optotype d'oeil gauche (101dL) est réglé pour être un multiple pair de la largeur (W), et
le moyen de commande d'affichage affiche le premier optotype d'oeil droit (101aR) et le premier optotype d'oeil gauche (101aL) de sorte que le degré d'écart dans la direction latérale entre les deux soit un multiple impair de la largeur (W) et affiche le second optotype d'oeil droit (101dR) et le second optotype d'oeil gauche (101dL) de sorte que le degré d'écart dans la direction latérale entre les deux soit un multiple impair de la largeur (W) et que le parallaxe de l'optotype de fusion (A101d) par rapport à l'optotype de référence (S101a) soit proche d'un parallaxe prévu, si le parallaxe de l'optotype de fusion (A101d) par rapport à la surface d'affichage est plus proche d'un parallaxe prévu lorsque le degré d'écart dans la direction latérale entre le second optotype d'oeil droit (101dR) et le second optotype d'oeil gauche (101dL) est réglé pour être un multiple pair de la largeur (W) que lorsque le degré d'écart dans la direction latérale entre le second optotype d'oeil droit (101dR) et le second optotype d'oeil gauche (101dL) est réglé pour être un multiple impair de la largeur (W).

3. Appareil de présentation d'optotype selon la revendication 1, comprenant en outre :
un moyen de réglage de distance (7) agencé pour entrer un signal de réglage d'une distance test entre la personne examinée et l'écran d'affichage ; et
une mémoire (21) qui stocke un modèle de l'optotype pour le test de vision stéréoscopique, la mémoire stockant au préalable, pour chacune des distances test qui peuvent être réglées par le moyen de réglage de distance, un premier modèle dans lequel le premier optotype d'oeil droit (101aR) et le premier optotype d'oeil gauche (101aL) sont affichés sans aucun degré d'écart dans la direction latérale entre eux, et le second optotype d'oeil droit (101dR) et le second optotype d'oeil gauche (101dL) sont affichés de sorte que le degré d'écart dans la direction latérale entre eux soit un multiple impair de la largeur (W) et que le parallaxe de l'optotype de fusion (A101d) par rapport à l'optotype de référence (S101a) soit proche d'un parallaxe prévu, et un second modèle dans lequel le premier optotype d'oeil droit (101aR) et le premier optotype d'oeil gauche (101aL) sont affichés de sorte que le degré d'écart dans la direction latérale entre eux soit un multiple impair de la largeur (W), et le second optotype d'oeil droit (101dR) et le second optotype d'oeil gauche (101dL) sont affichés de sorte que le degré d'écart dans la direction latérale entre eux soit un multiple impair de la largeur (W) et que le parallaxe de l'optotype de fusion (A101d) par rapport à l'optotype de référence (S101a) soit proche d'un parallaxe prévu,
dans lequel le moyen de commande d'affichage est agencé pour extraire le modèle stocké dans la mémoire sur la base de la distance test réglée par le moyen de réglage de distance et pour commander l'affichage afin d'afficher l'optotype selon le modèle extrait.
